# EUROPEAN PATENT APPLICATION

(11) **EP 4 732 830 A1**
(43) Date of publication of application: **29.04.2026**
(21) Application number: 25209741.5
(22) Date of filing: 20.10.2025
(51) Int. Cl.: A61K 9/00, A61K 9/12, A61K 47/14, A61K 31/565

(54) **VAGINAL FOAM FORMULATION AND PREPARATION METHOD THEREFOR**

(30) Priority: 24.10.2024 CN 202411496517
(71) Applicant: Guangdong Hongshanhu Pharmaceutical Co.,Ltd., Dongguan City, Guangdong Province 523000 (CN); Kunming Yuanrui Pharmaceutical Co., Ltd., Kunming City, Yunnan 651701 (CN)
(72) Inventor: WANG, Gaohua, Beijing, 100071 (CN); WU, Longhao, Beijing, 102400 (CN); LI, Pengfei, Beijing, 102400 (CN); ZHANG, Yanyuan, Beijing, 102400 (CN); LI, Juan, Beijing, 102400 (CN); XU, Chunxia, Beijing, 100071 (CN); LIANG, Yuhuan, Dongguan City, 523000 (CN)
(74) Representative: Morabito, Sara

(57) **Abstract**

Disclosed are a vaginal foam formulation and a preparation method therefor, which belong to the technical field of pharmaceuticals. Provided is a vaginal foam formulation with estradiol as an active ingredient. In the present disclosure, estradiol is prepared as a vaginal foam formulation, which exhibits good compliance, has a large contact area within the vagina, and allows for rapid release of estradiol. Therefore, the vaginal foam formulation has strong targeting and quick onset of action for diseases caused by estrogen deficiency, and features accurate administration dosage and convenient use.

## Description

### TECHNICAL FIELD

The present disclosure relates to the technical field of pharmaceuticals, and in particular relates to a vaginal foam formulation and a preparation method therefor.

### BACKGROUND

Estrogen plays a wide range of physiological roles in the female body. In addition to acting on the reproductive system, estrogen acts on other target organs, including bones, the cardiovascular system, the central nervous system, the liver, the skin and appendages thereof. Ovarian hypofunction caused by menopause or other factors can lead to estrogen deficiency, which in turn affects the function of these target organs, with specific manifestation in reproductive organs as atrophy of the vulva and vagina and reduced secretion. These conditions are not age-independent and the affected population often includes some young women. In addition to the need for estrogen supplementation due to normal function decline, there is also a portion of pathological needs for estrogen supplementation. The human body produces three types of estrogens, specifically estradiol, estrone and estriol. Estradiol is the most predominant and most active of the estrogens and is one of the six routine indicators of sex hormones. The structural formula of estradiol is as follows:

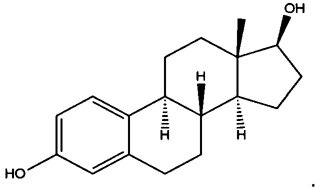

Currently, the more commonly used dosage forms and methods of administration of estradiol include: 1, injectable or oral estradiol (tablets, capsules, etc.), which has the drawbacks of large dosage specification, significant side effects (high incidence of breast cancer and cervical cancer) and low bioavailability; 2, topical estradiol (patches, films, etc.), which has the drawbacks of noticeable sensation of the patch or film adhering to the skin, poor compliance and a tendency to cause allergies in some patients; 3, estradiol ring or estradiol implant, where the estradiol ring has the drawbacks of relatively large volume, which causes a strong and uncomfortable foreign body sensation after implantation in the human body, resulting in poor compliance; the estradiol implant is relatively small, but still causes a noticeable foreign body sensation, and has the drawback of being prone to dislodgement; 4, estradiol vaginal tablet or estradiol vaginal soft capsule, which has the drawbacks of a noticeable foreign body sensation and poor compliance, and also features slow release of estradiol, resulting in little impact on cardiovascular-related symptoms, and being unfavorable for conditions highly dependent on local estrogen concentrations, such as sexual intercourse disorders, vaginal inflammation, and symptoms of vulvar atrophy requiring alleviation.

### SUMMARY

It is an objective of the present disclosure to provide a vaginal foam formulation and a preparation method therefor. In the present disclosure, estradiol is prepared as a vaginal foam formulation, which exhibits good compliance, has a large contact area within the vagina, and allows for rapid release of estradiol. Therefore, the vaginal foam formulation has strong targeting and quick onset of action for diseases caused by estrogen deficiency, and features accurate administration dosage and convenient use.

In order to achieve the above objective, the present disclosure provides the following technical solutions:
The present disclosure provides a vaginal foam formulation, which includes estradiol as an active ingredient.

Preferably, preparation raw materials for the vaginal foam formulation include a matrix material and a propellant, and preparation raw materials for the matrix material include estradiol, an oil-water amphoteric organic solvent, a hydrophobic ingredient, a surfactant and water.

Preferably, the matrix material includes, by mass percentage, the following preparation raw materials: 0.0001-0.01% of estradiol, 35-65% of an oil-water amphoteric organic solvent, 1.0-10% of a hydrophobic ingredient, and 0.1-4.0% of a surfactant, with the balance being water;
a usage amount of the propellant is 4-8 g/bottle, based on a usage amount of the matrix material being 10-100 g/bottle.

Preferably, the oil-water amphoteric organic solvent is an alcoholic solvent.

Preferably, the alcoholic solvent includes one or more of butylene glycol, propylene glycol, glycerol and polyethylene glycol.

Preferably, the hydrophobic ingredient includes one or more of C12-C22 fatty alcohol compounds, C12-C22 solid fatty acid ester compounds and an oily ingredient.

Preferably, the C12-C22 fatty alcohol compounds include cetyl alcohol and/or stearyl alcohol; the C12-C22 solid fatty acid ester compounds include one or more of glyceryl monostearate, glyceryl distearate and glyceryl mono- and distearate; the oily ingredient includes one or more of liquid fatty acid ester compounds, mineral oil and vegetable oil.

Preferably, the liquid fatty acid ester compounds include one or more of isopropyl myristate, isopropyl palmitate and ethyl oleate; the mineral oil includes one or more of liquid paraffin, petrolatum and white wax; the vegetable oil includes one or more of soybean oil, peanut oil and olive oil.

Preferably, the surfactant includes one or more of benzenesulfonate, fatty alcohol polyoxyethylene ether, alkylphenol polyoxyethylene ether, polyoxyethylene stearate, fatty amine polyoxyethylene ether, alkylolamide polyoxyethylene ether, lecithin, fatty acid monoglyceride, fatty acid diglyceride, sucrose ester of fatty acid, polysorbate, polyoxyethylene ricinoleate, ethoxylated glyceryl triricinoleate, diethylene glycol monoethyl ether, and triethanolamine.

Preferably, the propellant includes hydrofluoroalkane.

Preferably, the hydrofluoroalkane includes one or more of tetrafluoroethane, heptafluoropropane, and difluoroethane.

Preferably, a pH of the matrix material is 3.0-7.0.

The present disclosure provides a preparation method for the vaginal foam formulation described in the above technical solutions, the method including the following steps:
mixing estradiol, an oil-water amphoteric organic solvent, a surfactant and water to obtain an aqueous phase;
adding a hydrophobic ingredient as an oil phase to the aqueous phase, and performing an emulsification treatment to obtain a matrix material; and
filling a propellant into the matrix material to obtain the vaginal foam formulation.

The present disclosure provides a vaginal foam formulation comprising estradiol as an active ingredient. In the present disclosure, estradiol is prepared as a vaginal foam formulation, which exhibits good compliance, has a large contact area within the vagina, and allows for rapid release of estradiol. Therefore, the vaginal foam formulation has strong targeting and quick onset of action for diseases caused by estrogen deficiency, and features accurate administration dosage and convenient use.

### BRIEF DESCRIPTION OF THE DRAWINGS

FIG. 1 shows a photograph of an applicator used in the embodiments of the present disclosure;
FIG. 2 shows photographs of foam properties of various grades when evaluating foam properties in the examples of the present disclosure;
FIG. 3 shows a diagram of the test results of the packaging material suitability of the product in Example 43 of the present disclosure;
FIG. 4 shows a diagram of the test results of the packaging material suitability of the product in Example 44 of the present disclosure;
FIG. 5 shows a diagram of the test results of the packaging material suitability of the product in Example 45 of the present disclosure;
FIG. 6 shows dissolution curves of the estradiol foam formulation in Example 46 of the present disclosure, as well as the commercially available estradiol soft capsule and vaginal tablet;
FIG. 7 shows rabbit pharmacokinetic curves of the estradiol foam formulation in Example 46 of the present disclosure and the commercially available estradiol vaginal tablet;
FIG. 8 shows pathological diagrams of rabbit vaginal mucosal tissues treated with the estradiol foam formulation in Example 46 of the present disclosure.

### DETAILED DESCRIPTION OF THE EMBODIMENTS

The present disclosure provides a vaginal foam formulation, which includes estradiol as an active ingredient. In the present disclosure, estradiol is prepared as a vaginal foam formulation, which exhibits good compliance, has a large contact area within the vagina, and allows for rapid release of estradiol. Therefore, the vaginal foam formulation has strong targeting and quick onset of action for diseases caused by estrogen deficiency, and features accurate administration dosage and convenient use.

As one embodiment of the present disclosure, preparation raw materials for the vaginal foam formulation provided by the present disclosure may include a matrix material and a propellant, and preparation raw materials for the matrix material may include estradiol, an oil-water amphoteric organic solvent, a hydrophobic ingredient, a surfactant, and water.

In the present disclosure, unless otherwise specified, the raw materials used are commercially available products well known to those skilled in the art or are prepared by methods well known to those skilled in the art.

In the present disclosure, the matrix material preferably includes, by mass percentage, the following preparation raw materials: 0.0001-0.01% of estradiol, 35-65% of oil-water amphoteric organic solvent, 1.0-10% of hydrophobic ingredient, and 0.1-4.0% of surfactant, with the balance being water; a usage amount of the propellant is 4-8 g/bottle, based on a usage amount of the matrix material being 10-100 g/bottle.

In the present disclosure, the preparation raw materials for the matrix material preferably include, by mass percentage, 0.0001-0.01% of estradiol, which specifically may be 0.0001%, 0.0004%, 0.0008%, 0.001%, 0.004%, 0.008% or 0.01%.

In the present disclosure, the preparation raw materials for the matrix material preferably include, by mass percentage, 25-78% of oil-water amphoteric organic solvent, which specifically may be 25%, 30%, 35%, 40%, 45%, 50%, 55%, 60%, 65%, 70%, 75% or 78%. In the present disclosure, the oil-water amphoteric organic solvent is preferably an alcoholic solvent; the alcoholic solvent preferably includes one or more of butylene glycol, propylene glycol, glycerol and polyethylene glycol; the polyethylene glycol is preferably one or more of polyethylene glycol 200, polyethylene glycol 400 and polyethylene glycol 600, more preferably polyethylene glycol 200. In the present disclosure, the alcoholic solvent specifically may be butylene glycol, propylene glycol, glycerol or polyethylene glycol, or may be a mixture of propylene glycol and glycerol, or may be a mixture of glycerol and polyethylene glycol; when the alcoholic solvent is a mixture of propylene glycol and glycerol, a mass ratio of the propylene glycol to the glycerol is preferably 1 : 2-10, which specifically may be 1 : 2.9, 1 : 5.4 or 1 : 9.4, and in this case, a mass content of the alcoholic solvent in the matrix material is preferably 30-60%, which specifically may be 30%, 35%, 40%, 45%, 50%, 55% or 60%; when the alcoholic solvent is a mixture of glycerol and polyethylene glycol, a mass ratio of the glycerol to the polyethylene glycol is preferably 1 : 2.5-7.0, which specifically may be 1 : 2.9, 1 : 3.5 or 1 : 5.9, and in this case, a mass content of the alcoholic solvent in the matrix material is preferably 35-65%, which specifically may be 35%, 40%, 45%, 50%, 55%, 60% or 65%. In the present disclosure, the above type and usage amount of oil-water amphoteric organic solvent is preferably used, which helps to ensure smooth emulsification treatment, and enables the foam to have good foam formability, and exhibit good compatibility with both the propellant and the metered-dose applicator of the foam; in addition, the above type of oil-water amphoteric organic solvent can be used as a moisturizer or lubricant for cavity administration, to ensure that the foam has good efficacy.

In the present disclosure, the preparation raw materials for the matrix material preferably include 1.0-10% of a hydrophobic ingredient, which specifically may be 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5% or 10%. In the present disclosure, the hydrophobic ingredient preferably includes one or more of C12-C22 fatty alcohol compounds, C12-C22 solid fatty acid ester compounds and an oily ingredient, and more preferably a mixture of C12-C22 fatty alcohol compounds, C12-C22 solid fatty acid ester compounds and an oily ingredient, and in this case, a mass ratio of the C12-C22 fatty alcohol compounds, the C12-C22 solid fatty acid ester compounds to the oily ingredient is preferably 0.4-4 : 0.1-1 : 3-10, more preferably 0.8-3 : 0.3-0.8 : 4-9, further preferably 1-2.5 : 0.4-0.7 : 5-8, further more preferably 1.5-2 : 0.5-0.6 : 6-7.

In the present disclosure, the C12-C22 fatty alcohol compounds preferably include cetyl alcohol and/or stearyl alcohol, which specifically may be cetyl alcohol or stearyl alcohol, or a mixture of cetyl alcohol and stearyl alcohol; a mass ratio of the cetyl alcohol to the stearyl alcohol in the mixture of cetyl alcohol and stearyl alcohol is preferably 1-3 : 1-3, which specifically may be 1 : 1, 2 : 1, 1 : 2, 3 : 1 or 1 : 3. When the preparation raw materials for the matrix material include C12-C22 fatty alcohol compounds, a mass content of the C12-C22 fatty alcohol compounds in the preparation raw materials for the matrix material is preferably 0.4-3.0%, which specifically may be 0.4%, 0.8%, 1.0%, 1.33%, 1.5%, 1.62%, 1.85%, 2.0%, 2.5% or 3.0%. In the present disclosure, the above types and usage amounts of C12-C22 fatty alcohol compounds are preferably used, which can function to regulate the viscosity of the system, while acting as the backbone component of the foam to provide support.

In the present disclosure, the C12-C22 solid fatty acid ester compounds preferably include one or more of glyceryl monostearate, glyceryl distearate, and glyceryl mono- and distearate, more preferably glyceryl monostearate. When the preparation raw materials for the matrix material include C12-C22 solid fatty acid ester compounds, a mass content of the C12-C22 solid fatty acid ester compounds in the preparation raw materials for the matrix material is preferably 0.1-1.0%, which specifically may be 0.1%, 0.12%, 0.15%, 0.2%, 0.25%, 0.3%, 0.4%, 0.5%, 0.6%, 0.7%, 0.8%, 0.9% or 1.0%. In the present disclosure, the above types and usage amounts of C12-C22 solid fatty acid ester compounds are preferably used, which helps to ensure that the foam has good appearance properties, being smooth and fine-textured.

In the present disclosure, the oily ingredient preferably includes one or more of liquid fatty acid ester compounds, mineral oil and vegetable oil, more preferably liquid fatty acid ester compounds or mineral oil; the liquid fatty acid ester compound preferably includes one or more of isopropyl myristate, isopropyl palmitate, and ethyl oleate, more preferably isopropyl myristate; the mineral oil preferably includes one or more of liquid paraffin, petrolatum and white wax, more preferably liquid paraffin, where the liquid paraffin is preferably light liquid paraffin; the vegetable oil preferably includes one or more of soybean oil, olive oil and peanut oil. In the present disclosure, when the preparation raw materials for the matrix material include liquid fatty acid ester compounds, a mass content of the liquid fatty acid ester compounds in the preparation raw materials for the matrix material is preferably 1.0-10%, which specifically may be 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5% or 10%; when the preparation raw materials for the matrix material include mineral oil, a mass content of the mineral oil in the preparation raw materials for the matrix material is preferably 3.0-10%, which specifically may be 3.0%, 3.5%, 4.0%, 4.5%, 5.0%, 5.5%, 6.0%, 6.5%, 7.0%, 7.5%, 8.0%, 8.5%, 9.0%, 9.5% or 10%. In the present disclosure, the above types and usage amounts of liquid fatty acid ester compounds and mineral oil are preferably used, which helps to ensure that the foam has good appearance properties, being smooth and fine-textured.

In the present disclosure, the preparation raw materials for the matrix material preferably include, by mass percentage, 0.1-4.0% of a surfactant, which specifically may be 0.1%, 0.5%, 1.0%, 1.5%, 2.0%, 2.5%, 3.0%, 3.5% or 4.0%. In the present disclosure, the surfactant preferably includes one or more of benzenesulfonate, fatty alcohol polyoxyethylene ether, alkylphenol polyoxyethylene ether, polyoxyethylene stearate, fatty amine polyoxyethylene ether, alkylolamide polyoxyethylene ether, lecithin, fatty acid monoglyceride, fatty acid diglyceride, sucrose ester of fatty acid, polysorbate (Tween), polyoxyethylene ricinoleate, ethoxylated glyceryl triricinoleate, diethylene glycol monoethyl ether and triethanolamine, more preferably Tween; the Tween is preferably Tween 60. In the present disclosure, the above type and usage amount of surfactant is preferably used, which simultaneously has an emulsifying function, and exhibits good compatibility with a propellant, thus helping to ensure that the foam has good physical stability.

In the present disclosure, the pH of the matrix material is preferably 3.0-7.0, more preferably 3.0-5.0, which specifically may be 3.0, 3.5, 4.0, 4.5 or 5.0. In the present disclosure, a pH of the matrix material is preferably controlled within the above range, so as to ensure that the foam formulation is suitable for the physiological pH environment of the vagina. Moreover, during the preparation of the foam formulation, no delamination phenomenon is observed in the matrix material, which exhibits good compatibility with both the propellant and the metered-dose applicator of the foam. As shown in the test results in Examples 43-45, when the pressure-resistant aluminum canister was cut open and the coated inner wall of the aluminum canister was observed, no coating detachment phenomenon was found.

In the present disclosure, when the pH of the matrix material does not meet the above requirements, the preparation raw materials for the matrix material preferably further include a pH regulator, and a usage amount of the pH regulator is based on ensuring that the pH of the matrix material is 3.0-7.0. In the present disclosure, the pH regulator may be an acid agent or an alkali agent, and the acid agent is preferably lactic acid.

In the present disclosure, a usage amount of the propellant is 4-8 g/bottle, based on a usage amount of the matrix material being 10-100 g/bottle; Specifically, a usage amount of the matrix material may be 10 g/bottle, 20 g/bottle, 30 g/bottle, 40 g/bottle, 50 g/bottle, 60 g/bottle, 70 g/bottle, 80 g/bottle, 90 g/bottle, or 100 g/bottle; a usage amount of the propellant may be 4 g/bottle, 4.5 g/bottle, 5 g/bottle, 5.5 g/bottle, 6 g/bottle, 6.5 g/bottle, 7 g/bottle, 7.5 g/bottle or 8 g/bottle. In the present disclosure, the propellant preferably includes hydrofluoroalkane; The hydrofluoroalkane preferably includes one or more of tetrafluoroethane, heptafluoropropane and difluoroethane, which specifically may be tetrafluoroethane, heptafluoropropane or difluoroethane, more preferably tetrachloroethane. In the present disclosure, the above type and usage amount of propellant is preferably used, such that the foam of the prepared foam formulation has a smooth and fine-textured appearance. In terms of spreadability, aliphatic alkane is stronger than hydrofluoroalkane. However, since the foam formulation is for vaginal administration and reaches the cervical os through the applicator, excessive spreadability is detrimental to patient safety and tolerance. Therefore, hydrofluoroalkane is preferably used as the propellant.

As one embodiment of the present disclosure, one of the preferred formulations of the vaginal foam formulation of the present disclosure is as follows:
By mass percentage, the preparation raw materials for the matrix material include: estradiol, the content of which is 0.0004-0.001%, more preferably 0.001%; isopropyl myristate, the content of which is preferably 3-10%, more preferably 7%; cetyl alcohol, the content of which is preferably 0.2-2.0%, more preferably 1.33%; glyceryl monostearate, the content of which is preferably 0.1-1.0%, more preferably 0.20%; Tween 60, the content of which is preferably 0.3-4.0%, more preferably 2.0%; polyethylene glycol 200, the content of which is preferably of 35-55%, more preferably 37.78%; glycerin, the content of which is preferably 0-10%, more preferably 8.59%; lactic acid, the content of which is preferably 0.05-0.2%, more preferably 0.10%, with the balance being water; a usage amount of the propellant is preferably 4-5.5 g/bottle, more preferably 4.7 g/bottle, based on the amount of the matrix material being 10-100 g/bottle (e.g., 40 g/bottle).

The present disclosure provides a preparation method for the vaginal foam formulation described in the above technical solutions, the method including the following steps:
mixing estradiol, an oil-water amphoteric organic solvent, a surfactant and water to obtain an aqueous phase;
adding a hydrophobic ingredient as an oil phase to the aqueous phase, and performing an emulsification treatment to obtain a matrix material; and
filling a propellant into the matrix material to obtain the vaginal foam formulation.

In the present disclosure, estradiol, oil-water amphoteric organic solvent, surfactant and water are mixed to obtain an aqueous phase. In the present disclosure, the mixing is preferably performed under heating conditions, and the temperature of the heating is preferably 75-85°C, more preferably 80°C.

In the present disclosure, after obtaining an aqueous phase, a hydrophobic ingredient is added as an oil phase to the aqueous phase, and an emulsification treatment is performed to obtain a matrix material. In the present disclosure, the hydrophobic ingredient is preferably heated, and the temperature of the heating is preferably 75-85°C, more preferably 80°C. In the present disclosure, the hydrophobic ingredient is preferably heated and ensured to be in a molten state. In the present disclosure, the heated hydrophobic ingredient is preferably added as an oil phase to the heated aqueous phase under stirring or shearing conditions, and an emulsification treatment is performed. In the present disclosure, the temperature of the emulsification treatment is preferably 75-85°C, more preferably 80°C. The time of the emulsification treatment is preferably 5-15 min, more preferably 10 min, and the time of the emulsification treatment is counted from the completion of the addition of the oil phase. The emulsification treatment is preferably performed under stirring or shear conditions. In the present disclosure, after the emulsification treatment, the resulting material liquid is preferably cooled to room temperature under stirring or shearing conditions to obtain the matrix material. In the present disclosure, when the raw preparation materials for the matrix material preferably further include a pH regulator, the pH regulator is preferably added to the material liquid cooled to room temperature to obtain the matrix material in the present disclosure. The matrix material of the present disclosure is a white or colorless liquid with certain viscosity, which helps the foam agent to have sufficient retention time in the cavity and make full contact with the cavity.

After a matrix material is obtained, a propellant is filled into the matrix material to obtain the vaginal foam formulation in the present disclosure. In the present disclosure, the specific operation method for filling the propellant into the matrix material is not specially limited, and any method well known to those skilled in the art can be used.

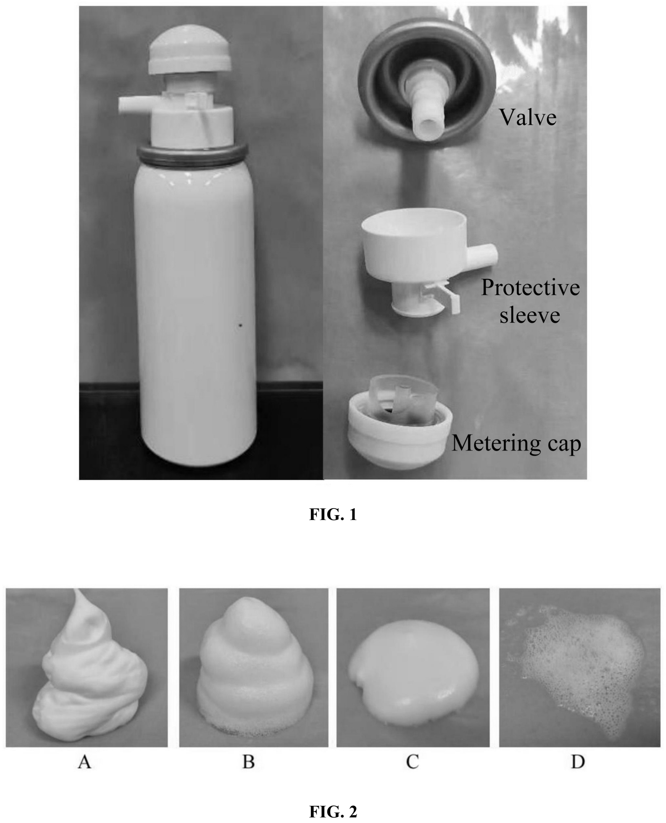

In the present disclosure, the vaginal foam formulation is preferably administered in a fixed dose; specifically, the vaginal foam formulation is administered to the vaginal cervical os through an applicator. FIG. 1 shows a photograph of an applicator used in the examples of the present disclosure, which preferably includes a pressure-resistant bottle, a valve, a protective sleeve, a metering cap and a catheter, where the catheter is specifically connected to a protruding site of the protective sleeve for insertion into the vagina for administration; the pressure-resistant bottle preferably includes a soda-lime glass, a borosilicate glass vial, a PC bottle or an aluminum canister, more preferably an aluminum canister; the valve is preferably a metering valve, where the specification of the metering valve is preferably 1 mL, that is, the volume of foam ejected by a single press of the metering cap is preferably 1 mL, and the mass of estradiol in 1 mL of the foam is preferably 4-10 µg, which specifically may be 4 µg, 5 µg, 6 µg, 7 µg, 8 µg, 9 µg or 10 µg; the catheter is preferably a disposable catheter. In the present disclosure, specifically, the matrix material is filled into a pressure-resistant bottle, a metering valve is pressed, and then a propellant is filled, and a protective sleeve and a metering cap are attached. In the present disclosure, the mass of the matrix material in each bottle of the vaginal foam formulation may be 40 g. For the sake of medication safety for the human body and environmental pollution impact, the propellant charge in each bottle of the vaginal foam formulation of the present disclosure may be 4-8 g, which specifically may be 4 g, 4.5 g, 5 g, 5.5 g, 6 g, 6.5 g, 7 g, 7.5 g or 8 g, and the pressure in each bottle of the vaginal foam formulation may be 6-8 bar, which specifically may be 6 bar, 6.5 bar, 7 bar, 7.5 bar or 8 bar. The vaginal foam formulation product provided by the present disclosure has a delivery uniformity result satisfying RSD ≤ 2%, which can achieve the purposes of quantitative administration and controllable quality.

The vaginal foam formulation provided by the present disclosure has a smooth and fine-textured foam appearance, and good stability. The vaginal foam formulation provided by the present disclosure is simple to use, clean and hygienic, is not limited to utensils and professional places, requires only a gentle press during use and the replacement of a simple catheter for each use, and has low cost; the vaginal foam formulation has good compliance, no foreign body sensation, a small amount of foam in the body is almost imperceptible; there is no need to worry about the dislodgement of the formulation, enabling normal daily life. The vaginal foam formulation provided by the present disclosure is expected to have good therapeutic efficacy, and the foam forms a drug-containing film layer on the surface of the cavity wall in the body, guaranteeing the time of administration; the vaginal foam formulation contains a propellant, which provides a certain impact pressure, enabling the formulation to fully expand to effectively fill and cover the deep vaginal area and folds, thus ensuring sufficient contact with the administration site; the vaginal foam formulation is packaged in a fully sealed high-pressure canister, which helps to improve product stability and prevent drug degradation; the foam formulation is light and silky, with complete cavity-filling ability, demonstrating a good application effect on conditions such as dyspareunia and dryness. The vaginal foam formulation provided by the present disclosure is safe and effective, with precise dosage and controllable quality.

The vaginal foam formulation provided by the present disclosure does not require the use of a large amount of surfactant or ethanol, thereby avoiding problems of skin irritation and dryness caused by the use of a large amount of ethanol in sprays; moreover, compared with the vaginal suppository, the vaginal foam formulation has a superior contact area with the vaginal mucosa, is convenient to administer, and does not require constant cleaning of the cavity. In addition, due to the targeted action of vaginal estrogen, the formulation does not require the addition of transdermal enhancers such as azone, which results in a simple composition, a simple preparation process, and convenient use, thereby enabling good patient compliance and adherence.

The technical solutions in the present disclosure will be described clearly and completely below with reference to the examples of the present disclosure. Apparently, the described examples are merely some, rather than all of the examples of the present disclosure. On the basis of the examples of the present disclosure, all other examples that can be obtained by those of ordinary skill in the art without creative efforts shall fall within the scope of protection of the present disclosure.

The estradiol formulations involved in the following examples and comparative examples include a matrix material and a propellant, where the matrix material specifically refers to the components in the estradiol formulations other than the propellant, the total mass of the matrix material per bottle of estradiol formulation is 40 g, the addition amounts of various components in the matrix material is calculated as a percentage of the total mass of the matrix material (i.e., the sum of the addition amounts of various components in the matrix material equals 100%), and the addition amount of the propellant is the addition mass of the propellant in each bottle of estradiol formulation.

The foam properties of the products in the following examples and comparative examples were evaluated, and specifically, the formulation quality was evaluated in terms of the properties of the ejected foam. FIG. 2 shows photographs of foam properties of various grades when evaluating foam properties, which are Grade A, Grade B, Grade C and Grade D from left to right. Among them, Grade A refers to foam that is well-formed, fine-textured and soft, and has good supporting force; Grade B refers to foam that is well-formed but not fine-textured, with slightly larger pores, and has slightly weaker supporting force; Grade C refers to a foamy state, but with no supporting force, presenting as reticulated foam; Grade D refers to a non-foamy state, existing in a liquid form.

The relative densities of the foams of the products in the following examples and comparative examples were determined. The specific method was as follows: Three samples were taken and placed at 25°C for 24 h, taking care to shake the vessel well but not heat the vessel, and discard 5-10 mL of foam. A flat weighing bottle having a volume of about 100 mL and a height of about 35 mm was prepared and accurately weighed, with mass recorded as W. Subsequently, the nozzle was placed at the bottom corner of the flat weighing bottle, and the metering cap was pressed to fill the flat weighing bottle with foam evenly in a circular motion. After the foam was completely expanded, the excess foam was flush with the upper edge of the flat weighing bottle using a spatula and carefully cleaned, and then the flat weighing bottle was accurately weighed, with the mass recorded as *W*₂. Subsequently, the flat weighing bottle was filled with purified water at 25°C and accurately weighed, with the mass recorded as W₁. The relative density was calculated as follows: ρ = (W₂ - W)/(W₁ - W).

The products in the following examples were investigated for high- and low-temperature physical stability. The specific method was as follows: The products were placed at low temperature (2-8°C) for 20 h, then returned to room temperature (25°C) and placed for 4 h, followed by placement at high temperature (30°C) for 20 h, and then returned to room temperature and placed for 4 h. This process constituted one cycle, and the experiment was performed for five consecutive cycles.

### Examples 1-4

Preparation of aqueous phase: A formulated amount of propylene glycol or polyethylene glycol 200 was weighed and mixed with a formulated amount of glycerol, Tween 60, estradiol and water. The mixture was heated to 80 ± 5°C until complete dissolution of the components, and then maintained at this temperature for later use.

Preparation of oil phase: A formulated amount of isopropyl myristate or light liquid paraffin was weighed and mixed with a formulated amount of glyceryl monostearate and cetyl alcohol. The mixture was heated to 80 ± 5°C until complete melting of the components.

Mixing: The oil phase was added to the aqueous phase under stirring. After the addition was completed, stirring was continued at 80 ± 5°C for 10 min. Subsequently, the resulting pharmaceutical liquid was cooled to room temperature under stirring, and the pH was adjusted to 3-5 with lactic acid to obtain a matrix material.

Filling: The matrix material was filled into an aluminum canister, a metering valve (1 mL) was pressed, and then tetrafluoroethane was filled, and a protective sleeve and a metering cap were attached.

The formulations of various products are shown in Table 1.

**Table 1 Formulations of products in Examples 1-4**

| Raw and auxiliary materials | | Example 1 | Example 2 | Example 3 | Example 4 |
|---|---|---|---|---|---|
| Mass percentage (%) of various components in the matrix material (40 g) | Estradiol | 0.0004 | 0.001 | 0.0004 | 0.001 |
| | Isopropyl myristate | 5.0 | 7.0 | / | / |
| | Glyceryl monostearate | 0.25 | 0.15 | 0.12 | 0.1 |
| | Light liquid paraffin | / | / | 5.0 | 7.0 |
| | Cetyl alcohol | 1.33 | 1.33 | 1.62 | 1.85 |
| | Tween 60 | 2 | 2 | 2 | 2 |
| | Propylene glycol | / | 42.15 | / | 43.55 |
| | Polyethylene glycol 200 | 37.78 | / | 39.0 | / |
| | Glycerol | 8.58 | 6.58 | 8.5 | 7.25 |
| | Lactic acid | 0.1 | 0.1 | 0.1 | 0.1 |
| | Water | Balance | Balance | Balance | Balance |
| Addition amount of tetrafluoroethane (g) | | 4.7 | 5.5 | 5.9 | 6.2 |

The quality of the formulations was evaluated in terms of the properties of the ejected foams, and the relative densities of the foams from formulations with good forming properties were determined. The specific test results are shown in Table 2. The results showed that when the mass percentage of estradiol in the matrix material was within the range of 0.0004-0.001%, good foams could be formed, which were fine-textured and intact, and all could persist in the ejected state.

**Table 2 Foam properties and relative densities of products in Examples 1-4**

| Test indicator | Example 1 | Example 2 | Example 3 | Example 4 |
|---|---|---|---|---|
| Property grade | A | A | A | A |
| Relative density | 0.1041 | 0.1039 | 0.1025 | 0.1011 |

### Examples 5-8 and Comparative Example 1

The procedures were performed with reference to the method of Example 1. The formulations of various products are shown in Table 3.

**Table 3 Formulations of products in Examples 5-8 and Comparative Example 1**

| Raw and auxiliary materials | | Example 5 | Example 6 | Example 7 | Example 8 | Comparative Example 1 |
|---|---|---|---|---|---|---|
| Mass percentage (%) of various components in the matrix material (40 g) | Estradiol | 0.001 | 0.001 | 0.001 | 0.001 | 0.001 |
| | Isopropyl myristate | 7 | 7 | 7 | 7 | 7 |
| | Glyceryl monostearate | 0.30 | 0.30 | 0.30 | 0.30 | 0.30 |
| | Cetyl alcohol | 1.2 | 1.2 | 1.2 | 1.2 | 1.2 |
| | Stearyl alcohol | 0.4 | 0.4 | 0.4 | 0.4 | 0.4 |
| | Tween 60 | 2 | 2 | 2 | 2 | 2 |
| | Propylene glycol | 25 | 50 | 20 | 70 | 10 |
| | Glycerol | 8.5 | 8.5 | 8.5 | 8.5 | 8.5 |
| | Lactic acid | 0.1 | 0.1 | 0.1 | 0.1 | 0.1 |
| | Water | Balance | Balance | Balance | Balance | Balance |
| Addition amount of tetrafluoroethane (g) | | 4.9 | 6.2 | 5.4 | 5.6 | 5.2 |

The quality of the formulations was evaluated in terms of the properties of the ejected foams, and the relative densities of the foams from formulations with good forming properties were determined. The specific test results are shown in Table 4. The results showed that when the total mass percentage of propylene glycol and glycerol in the matrix material was within the range of 30-60%, the foams had good formability, were fine-textured and intact, and could persist in the ejected state.

**Table 4 Foam properties and relative densities of products in Examples 5-8 and Comparative Example 1**

| Test indicator | Example 5 | Example 6 | Example 7 | Example 8 | Comparative Example 1 |
|---|---|---|---|---|---|
| Property grade | B | A | C | C | D |
| Relative density | 0.1003 | 0.1011 | 0.1056 | 0.1066 | / |

The products in Examples 5-6 were investigated for high- and low-temperature physical stability. The specific test results are shown in Table 5. The results showed that the products in Examples 5-6 had excellent high- and low-temperature physical stability.

**Table 5 Test results of high- and low-temperature physical stability of products in Examples 5-6**

| Experimental cycle | Storage condition | Evaluation item | Example 5 | Example 6 |
|---|---|---|---|---|
| 1 | 2-8°C | Foam property | B | A |
| | | Return to 25°C | B | A |
| | 30°C | Foam property | B | A |
| | | Return to 25°C | B | A |
| 2 | 2-8°C | Foam property | B | A |
| | | Return to 25°C | B | A |
| | 30°C | Foam property | B | A |
| | | Return to 25°C | B | A |
| 3 | 2-8°C | Foam property | B | A |
| | | Return to 25°C | B | A |
| | 30°C | Foam property | B | A |
| | | Return to 25°C | B | A |
| 4 | 2-8°C | Foam property | B | A |
| | | Return to 25°C | B | A |
| | 30°C | Foam property | B | A |
| | | Return to 25°C | B | A |
| 5 | 2-8°C | Foam property | B | A |
| | | Return to 25°C | B | A |
| | 30°C | Foam property | B | A |
| | | Return to 25°C | B | A |

The delivery of each puff was evaluated in terms of the ejected foam, primarily to investigate the uniformity of delivery per puff. Specifically, one canister of the sample was taken and shaken, and then the metering cap was pressed. The canister was kept vertically inverted for 10 s, and then returned to the upright position. The metering cap was released. The first puff was discarded, and a total of ten delivered doses were collected, including the initial three doses, the middle four doses, and the final three doses. The RSD values were calculated. The test results are shown in Table 6. The results showed that the RSD of delivery uniformity for the products in Examples 5-6 was less than 2%.

**Table 6 Test results of delivery uniformity for products in Examples 5-6**

| Examples | 1# (%) | 2# (%) | 3# (%) | 4# (%) | 5# (%) | 6# (%) | 7# (%) | 8# (%) | 9# (%) | 10# (%) | Average (%) | RSD (%) |
|---|---|---|---|---|---|---|---|---|---|---|---|---|
| 5 | 99.1 | 100.2 | 99.6 | 100.6 | 103.7 | 98.9 | 101.2 | 103.3 | 98.3 | 99.5 | 100.4 | 1.72 |
| 6 | 99.8 | 101.2 | 102.3 | 99.6 | 98.6 | 100.1 | 99.9 | 102.3 | 99.6 | 98.6 | 100.2 | 1.26 |

### Examples 9-12 and Comparative Examples 2-3

The procedures were performed with reference to the method of Example 1. The formulations of various products are shown in Table 7.

**Table 7 Formulations of products in Examples 9-12 and Comparative Examples 2-3**

| Raw and auxiliary materials | | Example 9 | Example 10 | Example 11 | Example 12 | Comparative Example 2 | Comparative Example 3 |
|---|---|---|---|---|---|---|---|
| Mass percentage (%) of various components in the matrix material (40 g) | Estradiol | 0.001 | 0.001 | 0.001 | 0.001 | 0.001 | 0.001 |
| | Isopropyl myristate | 7 | 7 | 7 | 7 | 7 | 7 |
| | Glyceryl monostearate | 0.2 | 0.2 | 0.2 | 0.2 | 0.2 | 0.2 |
| | Cetyl alcohol | 1 | 1 | 1 | 1 | 1 | 1 |
| | Stearyl alcohol | 0.5 | 0.5 | 0.5 | 0.5 | 0.5 | 0.5 |
| | Tween 60 | 2 | 2 | 2 | 2 | 2 | 2 |
| | Polyethylene glycol 200 | 30 | 40 | 50 | 25 | 20 | 57.5 |
| | Glycerol | 8.5 | 8.5 | 8.5 | 8.5 | 8.5 | 8.5 |
| | Lactic acid | 0.1 | 0.1 | 0.1 | 0.1 | 0.1 | 0.1 |
| | Water | Balance | Balance | Balance | Balance | Balance | Balance |
| Addition amount of tetrafluoroethane (g) | | 6.8 | 6.7 | 6.9 | 6.2 | 6.4 | 6.7 |

The quality of the formulations was evaluated in terms of the properties of the ejected foams, and the relative densities of the foams from formulations with good forming properties were determined. The specific test results are shown in Table 8. The results showed that when the total mass percentage of polyethylene glycol 200 and glycerol in the matrix material is within the range of 35-65%, the foams had good formability, were fine-textured and intact, and could persist in the ejected state.

**Table 8 Foam properties and relative densities of products in Examples 9-12 and Comparative Examples 2-3**

| Test indicator | Example 9 | Example 10 | Example 11 | Example 12 | Comparative Example 2 | Comparative Example 3 |
|---|---|---|---|---|---|---|
| Property grade | B | A | B | C | D | D |
| Relative density | 0.1002 | 0.1006 | 0.1009 | 0.1015 | / | / |

The products in Examples 9-11 were investigated for high- and low-temperature physical stability. The specific test results are shown in Table 9. The results showed that the products in Examples 9-11 had excellent high- and low-temperature physical stability.

**Table 9 Test results of high- and low-temperature physical stability of products in Examples 9-11**

| Experimental | Storage | Evaluation item | Example 9 | Example 10 | Example 11 |
|---|---|---|---|---|---|
| cycle | condition | | | | |
| 1 | 2-8°C | Foam property | B | A | B |
| | | Return to 25°C | B | A | B |
| | 30°C | Foam property | B | A | B |
| | | Return to 25°C | B | A | B |
| 2 | 2-8°C | Foam property | B | A | B |
| | | Return to 25°C | B | A | B |
| | 30°C | Foam property | B | A | B |
| | | Return to 25°C | B | A | B |
| 3 | 2-8°C | Foam property | B | A | B |
| | | Return to 25°C | B | A | B |
| | 30°C | Foam property | B | A | B |
| | | Return to 25°C | B | A | B |
| 4 | 2-8°C | Foam property | B | A | B |
| | | Return to 25°C | B | A | B |
| | 30°C | Foam property | B | A | B |
| | | Return to 25°C | B | A | B |
| 5 | 2-8°C | Foam property | B | A | B |
| | | Return to 25°C | B | A | B |
| | 30°C | Foam property | B | A | B |
| | | Return to 25°C | B | A | B |

### Examples 13-18

The procedures were performed with reference to the method of Example 1. The formulations of various products are shown in Table 10.

**Table 10 Formulations of products in Examples 13-18**

| Raw and auxiliary materials | | Example 13 | Example 14 | Example 15 | Example 16 | Example 17 | Example 18 |
|---|---|---|---|---|---|---|---|
| Mass percentage (%) of various components in the matrix material (40 g) | Estradiol | 0.001 | 0.001 | 0.001 | 0.001 | 0.001 | 0.001 |
| | Isopropyl myristate | 9.0 | 9.0 | 7.0 | 7.0 | 5.0 | 5.0 |
| | Glyceryl monostearate | 0.75 | 0.75 | 0.75 | 0.75 | 0.75 | 0.75 |
| | Cetyl alcohol | 0.4 | / | 1.2 | / | 2.0 | / |
| | Stearyl alcohol | / | 0.4 | / | 1.2 | / | 2.0 |
| | Tween 60 | 2.0 | 2.0 | 2.0 | 2.0 | 2.0 | 2.0 |
| | Polyethylene glycol 200 | 39 | 39 | 39 | 39 | 40 | 40 |
| | Glycerol | 9.5 | 9.5 | 8.5 | 8.5 | 7.5 | 7.5 |
| | Lactic acid | 0.1 | 0.1 | 0.1 | 0.1 | 0.1 | 0.1 |
| | Water | Balance | Balance | Balance | Balance | Balance | Balance |
| Addition amount of tetrafluoroethane (g) | | 6.7 | 6.8 | 7.2 | 6.7 | 7.3 | 6.4 |

The quality of the formulations was evaluated in terms of the properties of the ejected foams, and the relative densities of the foams were determined. The specific test results are shown in Table 11. The results showed that when cetyl alcohol or stearyl alcohol was used alone, and the mass percentage of cetyl alcohol or stearyl alcohol in the matrix material was within the range of 0.4-2%, the foams had good formability, were fine-textured and intact, and all could persist in the ejected state.

**Table 11 Foam properties and relative densities of products in Examples 13-18**

| Test indicator | Example 13 | Example 14 | Example 15 | Example 16 | Example 17 | Example 18 |
|---|---|---|---|---|---|---|
| Property grade | C | C | B | B | C | C |
| Relative density | / | / | 0.1023 | 0.1015 | 0.1003 | 0.1015 |

### Examples 19-23

The procedures were performed with reference to the method of Example 1. The formulations of various products are shown in Table 12.

**Table 12 Formulations of products in Examples 19-23**

| Raw and auxiliary materials | | Example 19 | Example 20 | Example 21 | Example 22 | Example 23 |
|---|---|---|---|---|---|---|
| Mass percentage (%) of various components in the matrix material (40 g) | Estradiol | 0.001 | 0.001 | 0.001 | 0.001 | 0.001 |
| | Isopropyl myristate | 7.0 | 5.0 | 5.0 | 3.0 | 3.0 |
| | Glyceryl monostearate | 0.95 | 0.35 | 0.35 | 0.25 | 0.25 |
| | Cetyl alcohol | 0.2 | 0.5 | 1.0 | 1.8 | 0.6 |
| | Stearyl alcohol | 0.2 | 1.0 | 0.5 | 0.6 | 1.8 |
| | Tween 60 | 2.0 | 2.0 | 2.0 | 2.0 | 2.0 |
| | Polyethylene glycol 200 | 39 | 39 | 39 | 39 | 39 |
| | Glycerol | 8.5 | 8.5 | 8.5 | 8.5 | 8.5 |
| | Lactic acid | 0.1 | 0.1 | 0.1 | 0.1 | 0.1 |
| | Water | Balance | Balance | Balance | Balance | Balance |
| Addition amount of tetrafluoroethane (g) | | 6.7 | 7.2 | 5.9 | 6.7 | 7.2 |

The quality of the formulations was evaluated in terms of the properties of the ejected foams, and the relative densities of the foams were determined. The specific test results are shown in Table 13. The results showed that when cetyl alcohol was used in combination with stearyl alcohol, the mass ratio of cetyl alcohol to stearyl alcohol was 1 : 1, 2 : 1, 1 : 2, 3 : 1, 1 : 3, and the total mass percentage of cetyl alcohol and stearyl alcohol in the matrix material was within the range of 0.4-3.0%, the components can function to adjust the viscosity of the system, while acting as the backbone components of the foam to provide support, and the foams had good formability, were fine-textured and intact, and could persist in the ejected state.

**Table 13 Foam properties and relative densities of products in Examples 19-23**

| Examples | Example 19 | Example 20 | Example 21 | Example 22 | Example 23 |
|---|---|---|---|---|---|
| Property | C | B | A | A | B |
| Relative density | 0.1002 | 0.1032 | 0.1027 | 0.1014 | 0.1008 |

The products in Example 19, Example 21 and Example 22 were investigated for high- and low-temperature physical stability. The specific test results are shown in Table 14. The results showed that the products in Example 19, Example 21 and Example 22 had good high- and low-temperature physical stability.

**Table 14 Test results of high- and low-temperature physical stability of the products in Example 19, Example 21 and Example 22**

| Experimental cycle | Storage condition | Evaluation item | Example 19 | Example 21 | Example 22 |
|---|---|---|---|---|---|
| 1 | 2-8°C | Foam property | C | A | A |
| | | Return to 25°C | C | A | A |
| | 30°C | Foam property | C | A | A |
| | | Return to 25°C | C | A | A |
| 2 | 2-8°C | Foam property | C | A | A |
| | | Return to 25°C | C | A | A |
| | 30°C | Foam property | C | A | A |
| | | Return to 25°C | C | A | A |
| 3 | 2-8°C | Foam property | C | A | A |
| | | Return to 25°C | C | A | A |
| | 30°C | Foam property | C | A | A |
| | | Return to 25°C | C | A | A |
| 4 | 2-8°C | Foam property | C | A | A |
| | | Return to 25°C | C | A | A |
| | 30°C | Foam property | C | A | A |
| | | Return to 25°C | C | A | A |
| 5 | 2-8°C | Foam property | C | A | A |
| | | Return to 25°C | C | A | A |
| | 30°C | Foam property | C | A | A |
| | | Return to 25°C | C | A | A |

### Examples 24-29

The procedures were performed with reference to the method of Example 1. The formulations of various products are shown in Table 15.

**Table 15 Formulations of products in Examples 24-29**

| Raw and auxiliary materials | | Example 24 | Example 25 | Example 26 | Example 27 | Example 28 | Example 29 |
|---|---|---|---|---|---|---|---|
| Mass percentage (%) of various components in the matrix material (40 g) | Estradiol | 0.001 | 0.001 | 0.001 | 0.001 | 0.001 | 0.001 |
| | Isopropyl myristate | 1.0 | 6.0 | 10.0 | / | / | / |
| | Glyceryl monostearate | 0.45 | 0.2 | 0.1 | 0.45 | 0.75 | 0.95 |
| | Light liquid paraffin | / | / | / | 2.0 | 7.0 | 10.0 |
| | Cetyl alcohol | 1.33 | 1.33 | 1.33 | 2.03 | 1.33 | 0.68 |
| | Tween | 2 | 2 | 2 | 2 | 2 | 2 |
| | Propylene glycol | 39 | 39 | 39 | 39 | 39 | 39 |
| | Glycerol | 8.5 | 8.5 | 8.5 | 8.5 | 8.5 | 8.5 |
| | Lactic acid | 0.1 | 0.1 | 0.1 | 0.1 | 0.1 | 0.1 |
| | Water | Balance | Balance | Balance | Balance | Balance | Balance |
| Addition amount of tetrafluoroethane (g) | | 4.9 | 5.2 | 5.6 | 5.7 | 6.2 | 5.6 |

The quality of the formulations was evaluated in terms of the properties of the ejected foams, and the relative densities of the foams were determined. The specific test results are shown in Table 16, The results showed that when the mass percentage of the hydrophobic ingredient fatty alcohol ester compound such as isopropyl myristate in the matrix material was within the range of 1.0-10%, the mass percentage of the light liquid paraffin was within the range of 2-10.0%, and the mass percentage of the glyceryl monostearate was within the range of 0.1-1.0%, the prepared foam formulations had good appearance properties, being smooth and fine-textured.

**Table 16 Foam properties and relative densities of products in Examples 24-29**

| Test indicator | Example 24 | Example 25 | Example 26 | Example 27 | Example 28 | Example 29 |
|---|---|---|---|---|---|---|
| Property grade | B | A | B | B | A | B |
| Relative density | 0.1012 | 0.1022 | 0.1012 | 0.1014 | 0.1014 | 0.1019 |

### Examples 30-34 and Comparative Examples 4-5

The procedures were performed with reference to the method of Example 1. The formulations of various products are shown in Table 17.

**Table 17 Formulations of products in Examples 30-34 and Comparative Examples 4-5**

| Raw and auxiliary materials | | Example 30 | Example 31 | Example 32 | Example 33 | Example 34 | Comparative Example 4 | Comparative Example 5 |
|---|---|---|---|---|---|---|---|---|
| Mass percentage (%) of various components in the matrix material (40 g) | Estradiol | 0.001 | 0.001 | 0.001 | 0.001 | 0.001 | 0.001 | 0.001 |
| | Isopropyl myristate | 5 | 5 | 5 | 5 | 5 | 5 | 5 |
| | Glyceryl monostearate | 0.15 | 0.15 | 0.15 | 0.15 | 0.15 | 0.15 | 0.15 |
| | Cetyl alcohol | 1.2 | 1.2 | 1.2 | 1.2 | 1.2 | 1.2 | 1.2 |
| | Stearyl alcohol | 0.6 | 0.6 | 0.6 | 0.6 | 0.6 | 0.6 | 0.6 |
| | Tween 60 | 0.3 | / | 4 | / | 5 | 0.1 | / |
| | S-40 ester | / | 0.3 | / | 4 | / | / | 0.1 |
| | Propylene glycol | 50 | 50 | 50 | 50 | 50 | 50 | 50 |
| | Glycerol | 9 | 9 | 9 | 9 | 9 | 9 | 9 |
| | Lactic acid | 0.1 | 0.1 | 0.1 | 0.1 | 0.1 | 0.1 | 0.1 |
| | Water | Balance | Balance | Balance | Balance | Balance | Balance | Balance |
| Addition amount of tetrafluoroethane (g) | | 6.7 | 6.5 | 4.9 | 5.2 | 6.4 | 5.6 | 5.8 |

The quality of the formulations was evaluated in terms of the properties of the ejected foams, and the relative densities of the foams were determined. The specific test results are shown in Table 18. The results showed that when the mass percentage of the emulsifier in the matrix material of the products in Comparative Examples 4-5 was 0.1%, the foams had poor appearance properties.

**Table 18 Foam properties and relative densities of products in Examples 30-34 and Comparative Examples 4-5**

| Examples | Example 30 | Example 31 | Example 32 | Example 33 | Example 34 | Comparative Example 4 | Comparative Example 5 |
|---|---|---|---|---|---|---|---|
| Property | B | B | B | B | C | D | D |
| Relative density | 0.1023 | 0.1002 | 0.1021 | 0.1001 | / | / | / |

The products in Examples 30-33 were investigated for high- and low-temperature physical stability. The specific test results are shown in Table 19. The results showed that when the mass percentage of the emulsifier in the matrix material was within the range of 0.3-4%, the prepared foam formulations had good appearance properties, being smooth and fine-textured.

**Table 19 Test results of high- and low-temperature physical stability of products in Examples 30-33**

| Experimental cycle | Storage condition | Evaluation item | Example 30 | Example 31 | Example 32 | Example 33 |
|---|---|---|---|---|---|---|
| 1 | 2-8°C | Foam property | B | B | B | B |
| | | Return to 25°C | B | B | B | B |
| | 30°C | Foam property | B | B | B | B |
| | | Return to 25°C | B | B | B | B |
| 2 | 2-8°C | Foam property | B | B | B | B |
| | | Return to 25°C | B | B | B | B |
| | 30°C | Foam property | B | B | B | B |
| | | Return to 25°C | B | B | B | B |
| 3 | 2-8°C | Foam property | B | B | B | B |
| | | Return to 25°C | B | B | B | B |
| | 30°C | Foam property | B | B | B | B |
| | | Return to 25°C | B | B | B | B |
| 4 | 2-8°C | Foam property | B | B | B | B |
| | | Return to 25°C | B | B | B | B |
| | 30°C | Foam property | B | B | B | B |
| | | Return to 25°C | B | B | B | B |
| 5 | 2-8°C | Foam property | B | B | B | B |
| | | Return to 25°C | B | B | B | B |
| | 30°C | Foam property | B | B | B | B |
| | | Return to 25°C | B | B | B | B |

### Examples 34-42

The procedures were performed with reference to the method of Example 1. The formulations of various products are shown in Table 20.

**Table 20 Formulations of products in Examples 34-42**

| Raw and auxiliary materials | | Exampl e 34 | Exampl e 35 | Exampl e 36 | Exampl e 37 | Exampl e 38 | Exampl e 39 | Exampl e 40 | Exampl e 41 | Exampl e 42 |
|---|---|---|---|---|---|---|---|---|---|---|
| Mass percentag e (%) of various componen ts in the matrix material (40 g) | Estradiol | 0.001 | 0.001 | 0.001 | 0.001 | 0.001 | 0.001 | 0.001 | 0.001 | 0.001 |
| | Isopropyl myristate | 2 | 2 | 2 | 7 | 7 | 7 | 10 | 10 | 10 |
| | Cetyl alcohol | 1.5 | 1.5 | 1.5 | 1.24 | 1.24 | 1.24 | 0.68 | 0.68 | 0.68 |
| | Stearyl alcohol | 0.3 | 0.3 | 0.3 | 0.41 | 0.41 | 0.41 | 0.24 | 0.24 | 0.24 |
| | Glyceryl monostearate | 0.2 | 0.2 | 0.2 | 0.15 | 0.15 | 0.15 | 0.32 | 0.32 | 0.32 |
| | Tween 60 | 2 | 2 | 2 | 2 | 2 | 2 | 2 | 2 | 2 |
| | PEG200 | 39 | 39 | 39 | 39 | 39 | 39 | 39 | 39 | 39 |
| | Glycerol | 8.5 | 8.5 | 8.5 | 8.5 | 8.5 | 8.5 | 8.5 | 8.5 | 8.5 |
| | Lactic acid | 0.1 | 0.1 | 0.1 | 0.1 | 0.1 | 0.1 | 0.1 | 0.1 | 0.1 |
| | Water | Balance | Balance | Balance | Balance | Balance | Balance | Balance | Balance | Balance |
| Addition amount of propellant (g) | Tetrafluoroethan e | 4.5 | / | / | 4.5 | / | / | 4.5 | / | / |
| | Difluoroethane | / | 5 | / | / | 5 | / | / | 5 | / |
| | Heptafluoroprop ane | / | / | 4.7 | / | / | 4.7 | / | / | 4.7 |

The quality of the formulations was evaluated in terms of the properties of the ejected foams, and the relative densities of the foams were determined. The specific test results are shown in Table 21. The results showed that there was no significant difference in the foam appearance among products prepared with different propellants, and the foams had smooth and fine-textured appearances. Tetrafluoroethane is preferably used as a propellant in the present disclosure.

**Table 21 Foam properties and relative densities of products in Examples 34-42**

| Test | Example | Example | Example | Example | Example | Example | Example | Example | Example |
|---|---|---|---|---|---|---|---|---|---|
| indicator | 34 | 35 | 36 | 37 | 38 | 39 | 40 | 41 | 42 |
| Property grade | B | B | B | A | A | A | B | B | B |
| Relative density | 0.1014 | 0.1025 | 0.1024 | 0.1014 | 0.1016 | 0.1044 | 0.1019 | 0.1044 | 0.1027 |

### Examples 43-45

The procedures were performed with reference to the method of Example 1. The formulations of the products are shown in Table 22.

**Table 22 Formulations of products in Examples 43-45**

| Raw and auxiliary materials | | Example 43 | Example 44 | Example 45 |
|---|---|---|---|---|
| Mass percentage (%) of various components in the matrix material (40 g) | Estradiol | 0.001 | 0.001 | 0.001 |
| | Isopropyl myristate | 7 | 7 | 7 |
| | Glyceryl monostearate | 0.2 | 0.2 | 0.2 |
| | Cetyl alcohol | 1.33 | 1.33 | 1.33 |
| | Tween 60 | 2 | 2 | 2 |
| | Polyethylene glycol 200 | 37.78 | 37.78 | 37.78 |
| | Glycerol | 8.59 | 8.59 | 8.59 |
| | Lactic acid | 0.17 | 0.11 | 0.067 |
| | Water | Balance | Balance | Balance |
| Addition amount of tetrafluoroethane (g) | | 4.92 | 4.85 | 4.96 |
| Product pH | | 3.10 | 4.22 | 4.95 |

The products in Examples 43-45 were investigated for stress stability at 40°C. Specifically, the products were placed at 40°C and samples were tested on day 0, day 10, and day 30. The changes in foam properties, pH, content, and related substances were investigated, and the packaging material suitability was observed at the different stages of the investigation.

The test methods for the content and related substances were as follows:
Determination method for content: The determination was performed by high-performance liquid chromatography (General Chapter 0512 of the Chinese Pharmacopoeia (2020 Edition, Volume IV)); chromatographic conditions: octadecylsilyl silica gel was used as the filler (Kromasil 4.6 mm × 250 mm, 5 µm, or chromatographic columns with equivalent efficiency); acetonitrile-water (45 : 55) was used as the mobile phase; the excitation wavelength was 240 nm, and the emission wavelength was 310 nm; the flow rate was 1 mL/min; the column temperature was 40°C; the injection volume was 20 µL.

Determination method for related substances: The determination was performed by high-performance liquid chromatography (General Chapter 0512 of the Chinese Pharmacopoeia (2020 Edition, Volume IV)); chromatographic conditions: octadecylsilyl silica gel was used as the filler (MN RP18 4.6 mm × 100 mm, 2.7 µm, or chromatographic columns with equivalent efficiency); linear gradient elution was performed with water as mobile phase A and acetonitrile as mobile phase B according to Table 23; the flow rate was 1 mL/min; the column temperature was 35°C; the detection wavelength was 220 nm; the injection volume was 10 µL.

**Table 23 Gradient elution method for related substances**

| Time (min) | Mobile phase A (%) | Mobile phase B (%) |
|---|---|---|
| 2 | 90 | 10 |
| 20 | 45 | 55 |
| 20.1 | 10 | 90 |
| 30 | 10 | 90 |
| 30.1 | 90 | 10 |
| 40 | 90 | 10 |

Result requirements: Total impurities ≤ 4.0%.

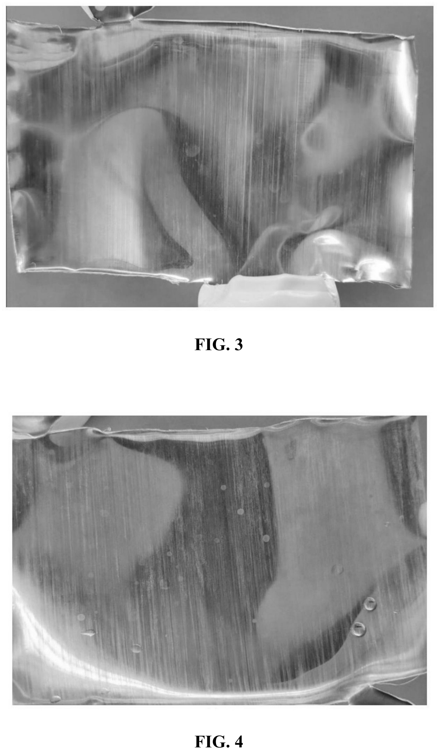

Table 24 shows the test results of stability at 40°C for the products in Examples 43-45. The results showed that after being placed at 40°C for 10 days, compared with the initial state on day 0, the foams of the products maintained supportive appearance properties, with accurate dosage of the ejected foam, and were smooth and fine-textured; the products showed no significant changes in the related substances and pH compared with the initial state on day 0, indicating that the samples exhibited excellent stability. After being placed at 40°C for 30 days, compared with the initial state on day 0, the products showed no significant changes in the content and related substances and had good compatibility with both the propellant and the metered-dose applicator of the foam formulation. When the pressure-resistant aluminum canister was cut open and the coated inner wall of the aluminum canister was observed, no coating detachment phenomenon was found (the coating condition of the aluminum canister after the product was stored at 40°C for 30 days is as shown in FIG. 3 to FIG. 5).

**Table 24 Test results of stability at 40°C for products in Examples 43-45**

| Sample source | | Test results of stress stability at 40°C | | |
|---|---|---|---|---|
| | | Day 0 | Day 10 | Day 30 |
| pH | Example 43 | 3.10 | 3.12 | 3.11 |
| | Example 44 | 4.22 | 4.25 | 4.17 |
| | Example 45 | 4.95 | 4.91 | 4.97 |
| Property | Example 43 | A | A | A |
| | Example 44 | A | A | A |
| | Example 45 | A | A | A |
| Content (%) | Example 43 | 99.56 | 99.27 | 99.17 |
| | Example 44 | 99.67 | 99.75 | 99.48 |
| | Example 45 | 99.62 | 99.46 | 99.64 |
| Related substances (%) | Example 43 | 1.0 | 1.24 | 1.42 |
| | Example 44 | 1.0 | 1.21 | 1.54 |
| | Example 45 | 1.0 | 1.19 | 1.36 |
| Packaging material suitability | Example 43 | No detachment was found | No detachment was found | No detachment was found |
| | Example 44 | No detachment was found | No detachment was found | No detachment was found |
| | Example 45 | No detachment was found | No detachment was found | No detachment was found |

### Example 46

The procedures were performed with reference to the method of Example 1. The formulations of the products are shown in Table 25.

**Table 25 Formulations of product in Example 46**

| Raw and auxiliary materials | | Example 46 |
|---|---|---|
| Mass percentage (%) of various components in the matrix material (40 g) | Estradiol | 0.001 |
| | Isopropyl myristate | 7 |
| | Glyceryl monostearate | 0.2 |
| | Cetyl alcohol | 1.33 |
| | Tween 60 | 2 |
| | Polyethylene glycol 200 | 37.78 |
| | Glycerol | 8.59 |
| | Lactic acid | 0.1 |
| | Water | Balance |
| Addition amount of tetrafluoroethane (g) | | 4.7 |

### Test example 1

The product in Example 46 was investigated for stress stability at 40°C, and subjected to a comparative study with the commercially available estradiol soft capsule and vaginal tablet. The specific test method and conditions refer to those in Example 43.

The determination results of the content are shown in Table 26. The results showed that the determination results of the content were within the range of 95-105%, and the products were qualified.

**Table 26 Test results of content**

| Sample source | Test results of stress stability at 40°C (unit: %) | | |
|---|---|---|---|
| | Day 0 | Day 10 | Day 30 |
| Example 46 | 99.5 | 98.63 | 98.27 |
| Vaginal tablet | 98.56 | 97.11 | 96.97 |
| Vaginal soft capsule | 99.32 | 97.2 | 96.61 |

The determination results of the related substances are shown in Table 27. The results showed that the change trend of the related substances of the product in Example 46 was better than that in the vaginal tablet and soft capsule.

**Table 27 Determination results of related substances**

| Sample source | Standard | Test results of stress stability at 40°C (unit: %) | | |
|---|---|---|---|---|
| | Total impurities ≤ 4.0% | Day 0 | Day 10 | Day 30 |
| Example 46 | Total impurities | 1.1 | 1.28 | 1.52 |
| Vaginal tablet | Total impurities | 1.0 | 2.8 | 3.6 |
| Soft capsule | Total impurities | 1.0 | 2.7 | 3.3 |

### Test example 2

The delivery of each puff was evaluated in terms of the ejected foam, primarily to investigate the uniformity of delivery per puff. Specifically, one canister of the sample was taken and shaken, and then the metering cap was pressed. The canister was kept vertically inverted for 10 s, and then returned to the upright position. The metering cap was released. The first puff was discarded, and a total of ten delivered doses were collected, including the initial three doses, the middle four doses, and the final three doses. The RSD values were calculated. The test results are shown in Table 28. The results showed that the RSD of delivery uniformity for the product in Example 46 was less than 2%.

**Table 28 Test results of delivery uniformity for product in Example 46**

| Examples | 1# (%) | 2# (%) | 3# (%) | 4# (%) | 5# (%) | 6# (%) | 7# (%) | 8# (%) | 9# (%) | 10# (%) | Average (%) | RSD (%) |
|---|---|---|---|---|---|---|---|---|---|---|---|---|
| 46 | 98.19 | 97.55 | 96.68 | 98.6 | 98.31 | 100.64 | 98.88 | 97.74 | 97.83 | 101.96 | 97.8 | 1.52 |

### Test example 3

The product in Example 46 was investigated for high- and low-temperature physical stability. The specific test results are shown in Table 29. The results showed that the foam formulation prepared in Example 46 had good appearance properties, being smooth and fine-textured.

**Table 29 Test results of high- and low-temperature physical stability of product in Example 46**

| Experimental cycle | Storage condition | Evaluation item | Example 46 |
|---|---|---|---|
| 1 | 2-8°C | Foam property | A |
| | | Return to 25°C | A |
| | 30°C | Foam property | A |
| | | Return to 25°C | A |
| 2 | 2-8°C | Foam property | A |
| | | Return to 25°C | A |
| | 30°C | Foam property | A |
| | | Return to 25°C | A |
| 3 | 2-8°C | Foam property | A |
| | | Return to 25°C | A |
| | 30°C | Foam property | A |
| | | Return to 25°C | A |
| 4 | 2-8°C | Foam property | A |
| | | Return to 25°C | A |
| | 30°C | Foam property | A |
| | | Return to 25°C | A |
| 5 | 2-8°C | Foam property | A |
| | | Return to 25°C | A |
| | 30°C | Foam property | A |
| | | Return to 25°C | A |

### Test example 4

The product in Example 46 was evaluated for *in-vitro* dissolution rate by using the basket method, and subjected to a comparative investigation on dissolution rate simultaneously with the commercially available estradiol soft capsule and vaginal tablet. The cumulative dissolution amount was determined by using the determination method for the content. The specific test conditions were as follows: The medium was 500 mL of phosphate buffer at pH 4.75, the rotation speed was 40 rpm, and the sampling time points were 1 h, 5 h, and 10 h, respectively.

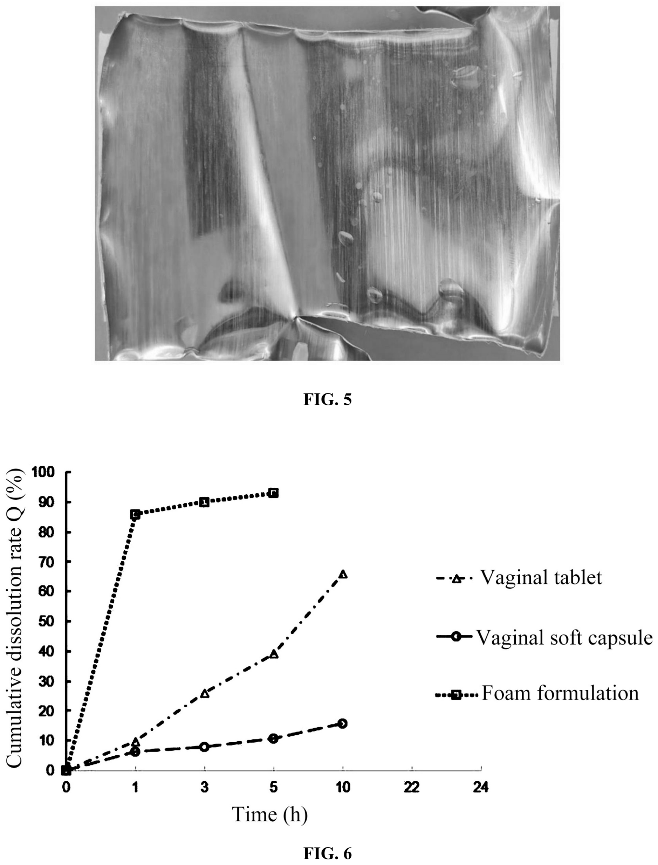

FIG. 6 shows dissolution curves of the product in Example 46, as well as the commercially available estradiol vaginal soft capsule and vaginal tablet.

The test results of the *in-vitro* dissolution rate are shown in Table 30. The results showed that the *in-vitro* release rate of the product in Example 46 was much faster at various time points than that of the vaginal tablet and soft capsule.

**Table 30 Test results of in-vitro dissolution rate**

| Sample source | Cumulative dissolution rate (%) | | | |
|---|---|---|---|---|
| | 1 h | 3 h | 5 h | 10h |
| Example 46 | 86 | 90 | 93 | / |
| Vaginal tablet | 9.7 | 26 | 39.1 | 65.8 |
| Vaginal soft capsule | 6.2 | 8.0 | 10.7 | 15.7 |

### Test example 5

Animal PK test: 14 SPF-grade New Zealand female rabbits (non-estrous cycle) weighing 2.0-2.5 kg were divided into groups T, R and P according to a (6 + 6 + 2) grouping strategy (where T represents test, R represents reference, and P represents control). Group T was given 1 puff and group R was given one formulation unit (1 tablet). 2 mL of blood was collected before the administration, and 1 h, 2 h, 4 h, 6 h, 8 h, 12 h and 24 h after the administration. The blood was left standing for serum separation, and then 0.3 mL of serum was transferred to a PE tube, which was stored in a refrigerator at -20°C for subsequent testing.

24 h after blood collection, the rabbits were sacrificed by air injection via the auricular vein. The vaginal tissues were taken, and the surrounding connective tissues were completely removed. The vagina was incised longitudinally, and the surface moisture was blotted with filter paper. First, the organs and vaginal inner walls were visually inspected for the presence of congestion, edema or other phenomena. Then, the vaginal tissues were placed in formalin solution for fixation for 24 h, followed by paraffin embedding, sectioning, and hematoxylin-eosin (HE) staining, and then subjected to histopathological examination and evaluation.

The content of estradiol in serum was determined by using the rabbit estradiol ELISA method, and the plasma concentrations measured by the test were subjected to mathematical statistics.

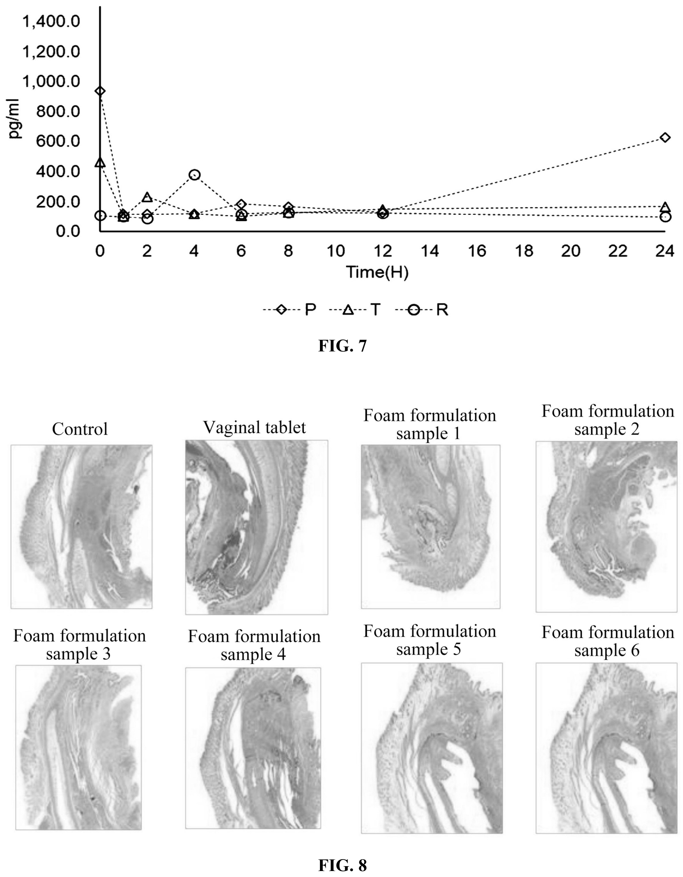

The results of the animal PK test on the test sample (product in Example 46) and the commercial estradiol vaginal tablet showed that after administration of the test sample and the commercially available estradiol vaginal tablet, no significant change in serum estradiol concentration was observed compared with the blank control group (Table 31, FIG. 7). In addition, after the administration, no erythema, edema or purulent discharge was observed in the periphery of the vaginal tissue, and the pathological sections of the vaginal mucosa showed no abnormalities (FIG. 8), indicating no mucosal irritation. These results suggested that the safety of the product was comparable to that of the reference.

**Table 31 In-vivo pharmacokinetic results**

| Time (h) | Group | 0 | 1 | 2 | 4 | 6 | 8 | 12 | 24 |
|---|---|---|---|---|---|---|---|---|---|
| Serum estradiol concentration (pg/mL) | P | 940.6 | 116.4 | 117.4 | 118.3 | 185.5 | 167.9 | 127.5 | 625.3 |
| | T | 464.2 | 99.5 | 232.1 | 119.3 | 104.2 | 124.7 | 149.4 | 165.8 |
| | R | 107.9 | 98.2 | 87.3 | 379.1 | 118.0 | 127.7 | 124.0 | 99.3 |

## Claims

1. A vaginal foam formulation, comprising estradiol as an active ingredient.

2. The vaginal foam formulation according to claim 1, wherein preparation raw materials for the vaginal foam formulation comprise a matrix material and a propellant, and preparation raw materials for the matrix material comprise estradiol, an oil-water amphoteric organic solvent, a hydrophobic ingredient, a surfactant and water.

3. The vaginal foam formulation according to claim 2, wherein the matrix material comprises, by mass percentage, the following preparation raw materials: 0.0001-0.01% of estradiol, 35-65% of oil-water amphoteric organic solvent, 1.0-10% of hydrophobic ingredient, and 0.1-4.0% of surfactant, with the balance being water; and
a usage amount of the propellant is 4-8 g/bottle, based on a usage amount of the matrix material being 10-100 g/bottle.

4. The vaginal foam formulation according to claim 2 or 3, wherein the oil-water amphoteric organic solvent is an alcoholic solvent.

5. The vaginal foam formulation according to claim 4, wherein the alcoholic solvent comprises one or more of butylene glycol, propylene glycol, glycerol, and polyethylene glycol.

6. The vaginal foam formulation according to claim 2 or 3, wherein the hydrophobic ingredient comprises one or more of C12-C22 fatty alcohol compounds, C12-C22 solid fatty acid ester compounds and an oily ingredient.

7. The vaginal foam formulation according to claim 6, wherein the C12-C22 fatty alcohol compounds comprise cetyl alcohol and/or stearyl alcohol; the C12-C22 solid fatty acid ester compounds comprise one or more of glyceryl monostearate, glyceryl distearate, and glyceryl mono- and distearate; the oily ingredient comprises one or more of liquid fatty acid ester compounds, mineral oil and vegetable oil.

8. The vaginal foam formulation according to claim 7, wherein the liquid fatty acid ester compounds comprise one or more of isopropyl myristate, isopropyl palmitate and ethyl oleate; the mineral oil comprises one or more of liquid paraffin, petrolatum and white wax; the vegetable oil comprises one or more of soybean oil, peanut oil, and olive oil.

9. The vaginal foam formulation according to claim 2 or 3, wherein the surfactant comprises one or more of benzenesulfonate, fatty alcohol polyoxyethylene ether, alkylphenol polyoxyethylene ether, polyoxyethylene stearate, fatty amine polyoxyethylene ether, alkylolamide polyoxyethylene ether, lecithin, fatty acid monoglyceride, fatty acid diglyceride, sucrose ester of fatty acid, polysorbate, polyoxyethylene ricinoleate, ethoxylated glyceryl triricinoleate, diethylene glycol monoethyl ether, and triethanolamine.

10. The vaginal foam formulation according to claim 2 or 3, wherein the propellant comprises hydrofluoroalkane.

11. The vaginal foam formulation according to claim 10, wherein the hydrofluoroalkane comprises one or more of tetrafluoroethane, heptafluoropropane and difluoroethane.

12. The vaginal foam formulation according to claim 2 or 3, wherein a pH of the matrix material is 3.0-7.0.

13. A method for preparing the vaginal foam formulation according to any one of claims 2-12, comprising the following steps:
mixing estradiol, an oil-water amphoteric organic solvent, a surfactant and water to obtain an aqueous phase;
adding a hydrophobic ingredient as an oil phase to the aqueous phase, and performing an emulsification treatment to obtain a matrix material; and
filling a propellant into the matrix material to obtain the vaginal foam formulation.

14. A foam formulation comprising estradiol as an active ingredient for use in the treatment of vagina.

15. The Foam formulation for use according to claim 1 or 14, wherein preparation raw materials for the vaginal foam formulation comprise a matrix material and a propellant, and preparation raw materials for the matrix material comprise estradiol, an oil-water amphoteric organic solvent, a hydrophobic ingredient, a surfactant and water; and
wherein the matrix material comprises, by mass percentage, the following preparation raw materials: 0.0001-0.01% of estradiol, 35-65% of oil-water amphoteric organic solvent, 1.0-10% of hydrophobic ingredient, and 0.1-4.0% of surfactant, with the balance being water; and
a usage amount of the propellant is 4-8 g/bottle, based on a usage amount of the matrix material being 10-100 g/bottle.
